# EUROPEAN PATENT APPLICATION

(11) **EP 4 778 579 A2**
(43) Date of publication of application: **22.07.2026**
(21) Application number: 26183305.7
(22) Date of filing: 04.10.2019
(51) Int. Cl.: A61P 13/12

(54) **METHODS TO TREAT RENAL DISORDERS USING CALCIUM CHANNEL INHIBITORS**

(30) Priority: 04.10.2018 US 201862741302 P; 19.07.2019 US 201962876186 P
(62) Divisional of application: 19870001.5
(71) Applicant: The Trustees of Indiana University, Bloomington, IN 47405-7000 (US)
(72) Inventor: BASILE, David, P., Indianapolis, 46236 (US); MEHROTRA, Purvi, Carmel, 46032 (US); STUREK, Michael, S., Indianapolis, 46202 (US)
(74) Representative: Pfenning, Meinig & Partner mbB

(57) **Abstract**

Disclosed are methods to treat a renal disorder in a mammal in need thereof by administering to the mammal in need of treatment an effective amount of a store operated calcium entry (SOCE) inhibitor or a pharmaceutically acceptable salt thereof.

## Description

### GOVERNMENT SUPPORT CLAUSE

This invention was made with government support under DK063114 awarded by National Institutes of Health. The government has certain rights in the invention.

### REFERENCE TO RELATED DISCLOSURES

The present application claims the benefit of U.S. Provisional Patent Application No. 62/741,302, filed Oct. 4, 2018, and U.S. Provisional Patent Application No. 62/876,186, filed July 19, 2019 both of which are incorporated herein by reference in their entirety.

### DESCRIPTION

Renal disease is a significant cause of adult mortality. Acute kidney injury (AKI) and chronic kidney disease (CKD) eventually progress to end-stage renal disease, requiring dialysis or kidney transplant. Unfortunately, no clinically proven therapies to prevent or to treat renal disease are available. A need in the art remains for methods to treat and to prevent renal disorders.

### BRIEF DESCRIPTION OF THE DRAWINGS

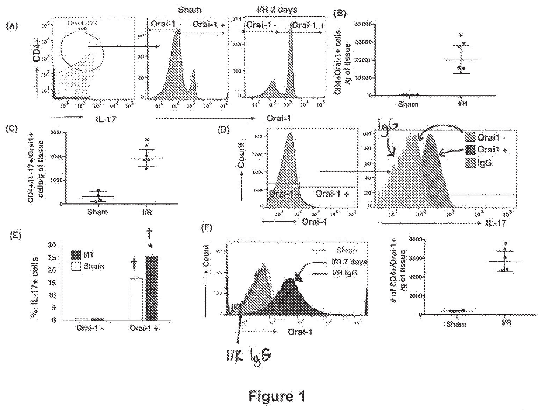

Figs. 1A-1F show co-expression of Orai1 and IL17 in CD4+ T cells following renal I/R Fig. 1A Left; Contour plot of renal lymphocytes stained with antibodies for CD4 and IL17 to identify Th17 cells. Orai1 staining in gated Th17 cells of sham is shown in middle panel and 2 days following I/R in the right panel. Fig. 1B) The number of Orai1+/CD4+cells in sham and post-I/R rat kidneys is shown. Fig. 1C) The number of Orai1+/CD4+/IL17+ cells in sham and post-I/R rat kidneys is shown. Fig. 1D) Representative histogram of Orai1+ cells in kidney CD4 fraction (left) 2 days following I/R and the distribution of IL17 expression as a function of Oria1 is shown on right. Fig. 1E) The percentage IL17+ cells as a function of Orai-1 expression in CD4+ cells following sham surgery or 2 days following I/R is shown Fig. 1F) Sustained expression of Orai1 in CD4+ cells following 7 days of recovery from I/R surgery is shown as representative histogram (left) and total number of Orai1+/CD4 cells is shown in the right panel. In Figs. B, C, E and F, data are mean ± SE of 4-5 rats per group; * indicates P < 0.05 sham vs post-AKI by Students t-test. In panel E, * indicates P < 0.05 in sham vs. I/R, † indicates P < 0.05 in Orai1- vs Orai1+ cells, by one-way ANOVA and Tukey's post-hoc test.

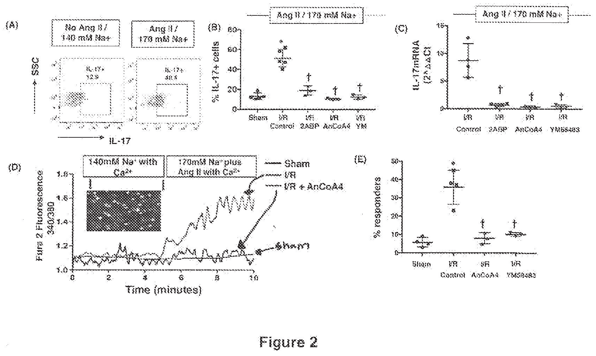

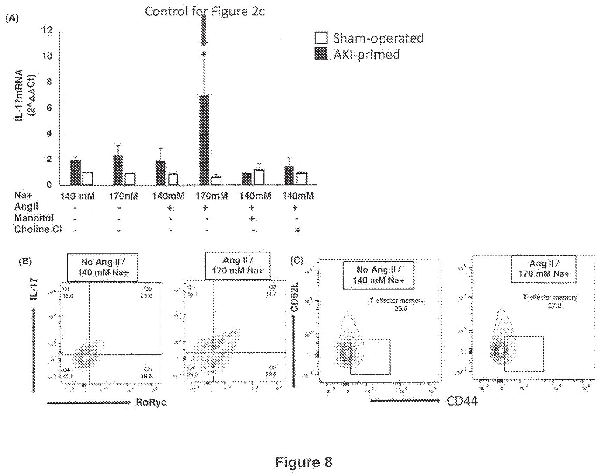

Figs. 2A-2E show Orai1 activity contributes to IL17 expression in CD4+ lymphocytes primed by renal ischemia/reperfusion injury. Fig. 2A) Illustrates representative FACS showing increased IL-17 expression in CD4+ cells from 7 day post-AKI rats following stimulation in vitro with 170 mM Na+ and Ang II vs. control media; Fig. 2B) percent IL17+ cells in CD4+ cells isolated 7 days following sham or AKI and stimulated in vitro; Fig. 2C) IL17 mRNA, expressed as 2^{-ΔΔcT} of kidney derived CD4+ cells isolated 7 days following I/R surgery and stimulated in vitro. In panel B and C, control refers to AKI-primed CD4+ cells stimulated with 170 mM Na⁺ and Ang II (10⁻⁷M), and SOCE inhibitors included as labeled. Fig. 2D) Fura-2 fluorescence imaging of intracellular Ca²⁺ in CD4+ lymphocytes in response to increased Na+ (170 mM) plus Ang II (10⁻⁷M), as indicated in the timeline and expressed as the ratio of fluorescence using 340/380 nm excitation. Shown are representative tracings of CD4+ cells from kidney following sham surgery (black) or I/R injury (red), or from I/R injury with co-incubation with AnCoA4 (blue). The inset illustrates representative visual field of multiple fura-2 loaded cells. Fig. 2E) Percent of cells manifesting an increase Ca²⁺ response relative to baseline following in vitro stimulation with increased Na/Ang II. Data are mean ± SE from 4-5 rats per group per assay; * indicates P < 0.05 vs unstimulated cells (i.e., no Ang II and normal Na, data not shown, see Fig. 8); † indicates P < 0.05 inhibitors vs stimulated post-AKI cells by one-ANOVA and Tukey's post-hoc test.

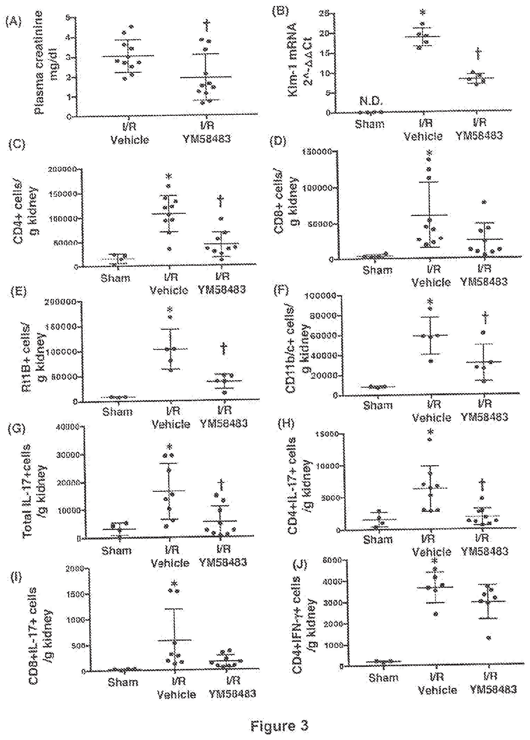

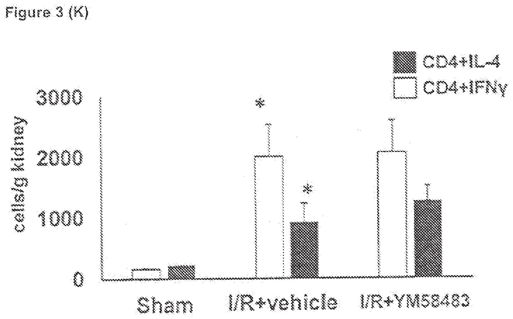

Figs. 3A-3K show YM58483 pretreatment attenuates renal ischemia reperfusion injury. Fig. 3A) Assessment of renal function by plasma creatinine 24 hours following I/R in Sprague-Dawley rats pretreated with YM-58483 or vehicle. Values for sham rats were 0.4 ± 0.1 mg/dl are not shown on graph. Fig. 3B) Kim-1 mRNA levels from total kidney RNA 24 hours following sham or I/R are shown, (N.D. = not detectable). Total kidney derived CD4+ and CD8+ cells are shown in Figs. 3C and 3D, respectively; total B cells (RT1B+), dendritic/macrophage cells (DC/M^{ϕ}; CD11b/c+) are shown in Figs. 3E and 3F, respectively. The total number IL17+, CD4+/IL17+ and CD8+/IL17+ cells are shown in Figs. 3G, 3H, 3I respectively. CD4+/IFNγ+ cells are shown in Fig. 3J and Fig. 3k. Data are mean ± S.E. (N=11rats/group in panel Fig. 3A and 7-8 rats in Figs. 3B-3J). *P<0.05 I/R vehicle vs sham; † P<0.05 vehicle vs YM58483 by one way ANOVA and Tukey's post-hoc test.

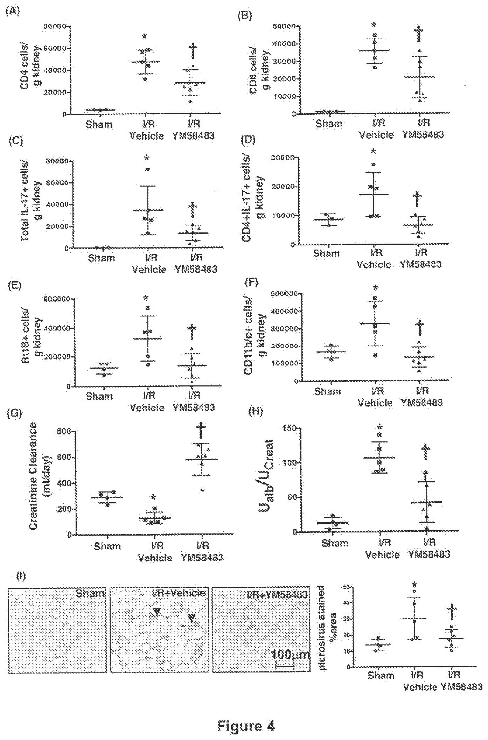

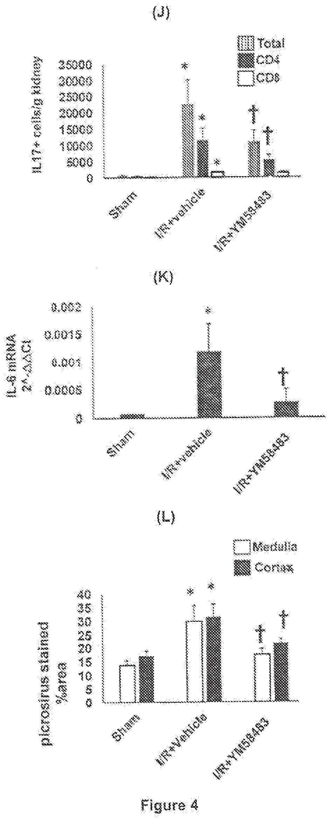

Figs. 4A-4L show levels of renal CD4+ (Fig. 4A), CD8+ T cells (Fig. 4B), IL-17+ expressing cells (Fig. 4C, and 4J), and CD4+/IL17+ cells (Fig. 4D). Levels of B cells (Fig. 4E) and dendritic cells/M^{ϕ} (Fig. 4F) are shown. Creatinine clearance from 24 hour urine collections at day 62-63 is shown in panel Fig. 4G and urinary albumin/creatinine ratio is shown in panel Fig. 4H. Panel Fig. 4I illustrates representative picrosirius red stained sections through renal outer medulla from sham, I/R vehicle or I/R + YM 58483-treated rats. Quantification of stained area is illustrated in Figs 4I and 4L. Kidney IL6 mRNA expression was also elevated in vehicle compared with sham, and was significantly attenuated by YM58483/BPT2 (Figure 4K). Data are mean ± S.E. (N=5-6 rats/group). *P<0.05 I/R vehicle vs sham; † P<0.05 vehicle vs YM58483 by one way ANOVA and Tukey's post-hoc test.

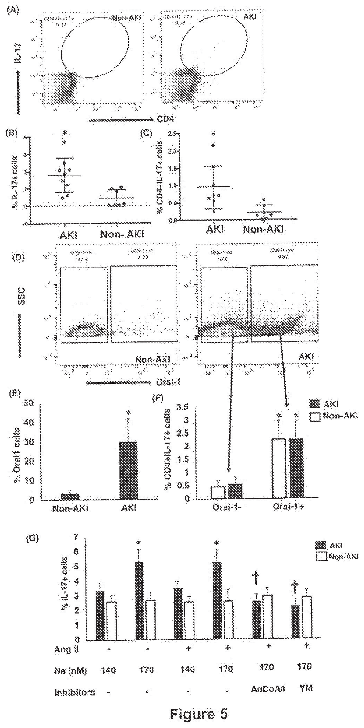

Figs. 5A-5G show elevated Th17 and Orai1 expression and effect of SOCE on IL17 responses in CD4+ blood lymphocytes in critical ill patients with vs without AKI. Fig. 5A) Representative FACS analysis of total peripheral blood cells in non-AKI (left, n=8) vs AKI patients (right, n=9) demonstrating the increase in CD4+IL17+ expressing cells. Quantification of the percentages of total IL17+ cells (Fig. 5B) and CD4+/IL17+ cells (Fig. 5C) in AKI vs non-AKI patients are shown. Representative dot plot showing an increase in Orai-1+ cells in AKI patients as compared to non-AKI patients is shown in (Fig. 5D) and the percentage of Orai-1+ cells in AKI vs non-AKI patients is shown in (Fig. 5E). (Fig. 5F) The %CD4+/IL17+ gated on Orai1+ and Orai1- fraction (arrows) is shown. In panel Fig. 5G, IL17 response to stimulation with elevated sodium (170 mM) and Ang II is shown for CD4+ cells isolated from AKI (n=5) or non-AKI (n=5) patients. Data are expressed as mean ± S.E. In panel Fig. 5B, Fig. 5C and Fig. 5D * indicates P<0.05 in AKI vs non-AKI patients by Student's t-test. In panel Fig. 5F, * indicates p<0.05 in Orai1+ vs Orai1- cells. In Fig 5G, * indicates p< 0.05 vs unstimulated (i.e., 140 mM and no Ang II), † p<0.05 inhibitors vs stimulated using one-way ANOVA and Tukey's post-hoc test.

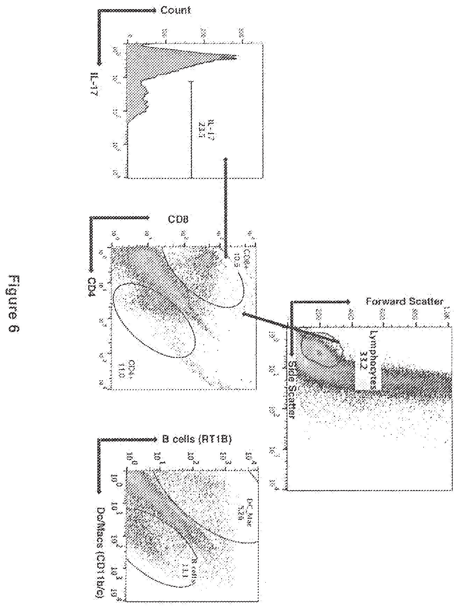

Fig. 6 shows gating strategies for the phenotypic analysis of infiltrating immune cells in the kidney. Lymphocyte gating is based on the forward scatter vs side scatter, which is further gated on CD4+ or CD8+T cells or B-cells or DC/M^{ϕ}. These populations were analyzed further based on IL-17 or Orai1 expression as described in text. B) Expression of Orai1 and Orai1+/IL17+ cells in CD8, B cells, NK cells and macrophages.

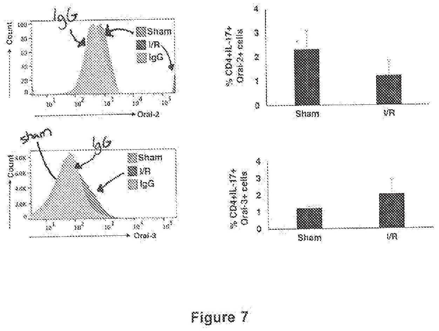

Fig. 7 shows Orai2 and Orai3 expression in kidney lymphocytes following renal I/R injury. A) Representative histogram of Orai2+ lymphocytes (left panel) and percent CD4+/Orai2+ cells in kidney 2 days following sham or I/R injury. B) Representative histogram of Orai3+ lymphocytes (left panel) and percent CD4+/Orai3+ cells in kidney 2 days following sham or I/R injury. Data are mean ±SE from a minimum of 3 independent rats per group; no statistical differences were observed between sham and I/R.
Figs. 8A - 8C show characterization of the cells used for in vitro stimulation following AKI priming. Fig. 8A) Representative FACS analysis of RORγT staining in CD4+ cells isolated 7 days following sham or I/R surgery. Fig. 8B) Renal injury primes IL17 mRNA response in kidney derived CD4+ cells. Renal CD4 cells were isolated from kidney 7 days following sham (open bar) or I/R surgery (black bar). Cells were incubated for 12-14 hours in media containing either 140 or 170 mM Na+ with or without Ang II (10-7M) as shown. To control for supplementation of NaCl to the media, some samples were stimulated with equimolar mannitol (60 mM) or choline chloride (30 mM) as shown. IL17 mRNA is expressed as 2-ΔΔCT and is mean ±SE from a minimum of 3 independent rats per group; * indicates P < 0.05 vs control (i.e., 140 mM Na+, no added Ang II), by one-ANOVA and Tukey's post-hoc test. Note the response of AKI primed cells with Ang II and added Na+ indicated by the arrow represent the control condition used in Figure 2C. Fig. 8C) Representative contour plots indicating the co-expression of IL17 with RORγT (RoRyc) in CD4+ cells from post-AKI rat kidney under control conditions and following stimulation with Ang II and elevated Na. D) Representative FACS analysis of CD4+ cells from post-IR rat kidney illustrating central memory T cells (CD44+CD62L-) before and after in vitro stimulation.

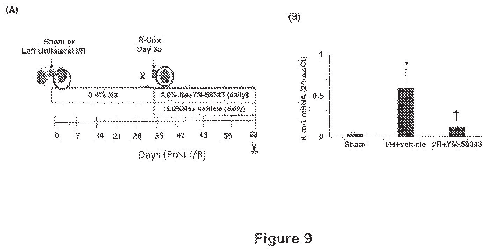

Figs. 9A-9B. Fig. 9A) Schematic outline of timeline to investigate the role of SOCE in progression of CKD following acute I/R injury. Fig. 9B) Renal Kim-1 expression measured in sham, I/R vehicle and YM 58483 is shown.

The invention provides store operated calcium entry (SOCE) inhibitors to treat a renal disorder in a mammal in need of such treatment, comprising administering to the mammal in need of treatment an effective amount of a SOCE inhibitor or a pharmaceutically acceptable salt thereof.

The invention further provides a pharmaceutical composition, comprising a SOCE inhibitor, or a pharmaceutically acceptable salt thereof, with one or more pharmaceutically acceptable carriers, diluents, or excipients.

The invention further provides a process for preparing a pharmaceutical composition, comprising admixing a SOCE inhibitor, or a pharmaceutically acceptable salt thereof, with one or more pharmaceutically acceptable carriers, diluents, or excipients.

This invention also provides a method to inhibit differentiation of a CD4+ cell to a T-helper 17 (TH17) cell, comprising administering to a mammal an effective amount of a store operated calcium entry SOCE inhibitor or a pharmaceutically acceptable salt thereof.

The invention additionally provides a method to modulate store operated calcium²⁺ entry into a cell, the method comprising administering to a mammal an effective amount of a store operated calcium entry SOCE inhibitor or a pharmaceutically acceptable salt thereof.

The invention further provides a method to decrease an amount of pro-inflammatory cytokine Interleuken 17 (IL-17), the method comprising administering to a mammal an effective amount of a store operated calcium entry SOCE inhibitor or a pharmaceutically acceptable salt thereof.

The invention also provides a method to decrease an amount of a Ca²⁺ release-activated Ca²⁺ channel pore forming subunit Oral1, the method comprising administering to a mammal an effective amount of a store operated calcium entry SOCE inhibitor or a pharmaceutically acceptable salt thereof.

The invention provides store operated calcium entry SOCE inhibitors to treat a renal disorder in a mammal in need of such treatment, comprising administering to the mammal in need of treatment an effective amount of a SOCE inhibitor or a pharmaceutically acceptable salt thereof. The SOCE inhibitors of the invention are any compound that interferes with the mechanism by which release of calcium ions from intracellular stores is coordinated with ion influx across the plasma membrane. In some embodiments, the SOCE inhibitor is selective for SOC channels and does not substantially affect the activity of other types of ion channels. In other embodiments, the SOCE inhibitor is selective for CRAC channels and does not substantially affect the activity of other types of ion channels and/or other SOC channels.

Exemplary SOCE inhibitors include, but are not limited to, 2-aminoethoxydiphenyl borate (2APB), (*N*-[4-[3,5-*Bis*(trifluoromethyl)-1*H*-pyrazol-1-yl]phenyl]-4-methyl-1,2,3-thiadiazole-5-carboxamide (YM58483/BTP2), AnCoA4, CM4620, GSK5948A, Synta66 and Oria1+ Si RNA.

The SOCE inhibitors of the invention are preferably formulated as pharmaceutical compositions administered by any route which makes the compound bioavailable, including oral and transdermal routes. Most preferably, such compositions are for oral administration. Such pharmaceutical compositions and processes for preparing same are well known in the art (See, e.g., Remington: The Science and Practice of Pharmacy, L.V. Allen, Editor, 22nd Edition, Pharmaceutical Press, 2012). The SOCE inhibitors, or pharmaceutically acceptable salts thereof are particularly useful in the treatment methods of the invention.

The SOCE inhibitors of the invention can be provided as a pharmaceutically acceptable salt. A pharmaceutically acceptable salt of the SOCE inhibitors of the invention can be formed, for example, by reaction of an appropriate free base of a compound of the invention and an appropriate pharmaceutically acceptable acid in a suitable solvent under standard conditions well known in the art. The formation of such salts is well known and appreciated in the art. See, for example, Gould, P.L., "Salt selection for basic drugs," International Journal of Pharmaceutics, 33: 201-217 (1986); Bastin, R.J., et al. "Salt Selection and Optimization Procedures for Pharmaceutical New Chemical Entities," Organic Process Research and Development, 4: 427-435 (2000); and Berge, S.M., et al., "Pharmaceutical Salts," Journal of Pharmaceutical Sciences, 66: 1-19, (1977).

The compounds of the present invention, or salts thereof, may be prepared by a variety of procedures known to one of ordinary skill in the art, some of which are illustrated in the schemes, preparations, and examples below. One of ordinary skill in the art recognizes that the specific synthetic steps for each of the routes described may be combined in different ways, or in conjunction with steps from different schemes, to prepare compounds of the invention, or salts thereof. The products of each step in the schemes below can be recovered by conventional methods well known in the art, including extraction, evaporation, precipitation, chromatography, filtration, trituration, and crystallization. In the schemes below, all substituents unless otherwise indicated, are as previously defined. The reagents and starting materials are readily available to one of ordinary skill in the art. Others may be made by standard techniques of organic and heterocyclic chemistry, which are analogous to the syntheses of known structurally similar compounds, and the procedures described herein which follow including any novel procedures. In addition, one of ordinary skill in the art appreciates that myriad SOCE inhibitors are commercially available and can therefore be readily obtained by one of skill in the art.

Renal disorders include, but are not limited to, acute kidney injury, chronic kidney disease and end stage renal disease. Acute kidney injury results from events such as renal ischemia, nephrotoxicity and/or sepsis. Renal disorders include renal inflammation, renal interstitial fibrosis, impaired renal function, proteinuria, and hypertension. In embodiments the renal disorder is renal inflammatory disorder and the disorder is ANCA associated vasculitis, crescentic glomerular nephritis, and nephrotic syndrome. In embodiments of the invention, the renal disorder is chronic allograft nephropathy in a kidney transplant recipient.

The terms "treating" or "to treat" include restraining, slowing, stopping, or reversing the progression or severity of an existing symptom or disorder. As used herein, the term "patient" refers to a human. The term "effective amount" refers to the amount or dose of compound of the invention, or a pharmaceutically acceptable salt thereof which, upon single or multiple dose administration to the patient, provides the desired effect in the patient under diagnosis or treatment.

An effective amount can be readily determined by one skilled in the art by the use of known techniques and by observing results obtained under analogous circumstances. In determining the effective amount for a patient, a number of factors are considered, including, but not limited to: the species of patient; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

The SOCE inhibitors are generally effective over a wide dosage range. For example, dosages per day normally fall within the range of about 0.5 µg/kg to about 30 mg/kg of body weight, preferably 0.5 mg/kg to about 10 mg/kg of body weight, more preferably 1.0 mg/kg to about 1.6 mg/kg of body weight. In some instances dosage levels below the lower limit of the aforesaid range may be more than adequate, while in other cases still larger doses may be employed with acceptable side effects, and therefore the above dosage range is not intended to limit the scope of the invention in any way.

The term "inhibiting" refers to slowing, decreasing, delaying, preventing or abolishing.

The term "differentiation" refers to change from relatively generalized to specialized kinds during development.

Acute kidney injury (AKI) results from events such as renal ischemia, nephrotoxicity and/or sepsis. AKI increases the risk of death in the intensive care unit (ICU), and mortality rates in this setting range between 15-60%. Survival from AKI is dependent on recovery of renal function following injury, the success of which has been suggested to be dependent on the efficiency of adaptive repair processes. Progression of chronic kidney disease (CKD) and end-stage kidney disease is recognized as a possible outcome of AKI patients, and it has been suggested that incomplete or maladaptive repair may predispose progression of CKD following AKI.

Immune cell activity may contribute to renal injury or may enhance renal recovery. In the setting of renal ischemia reperfusion (I/R) injury, renal CD4+ T-helper 1 or 17 cells are thought to exacerbate renal injury while T-regulatory cells have been implicated in renal repair (6, 7). Following recovery from I/R injury in rats, subsequent exposure to high-salt diet was shown to hasten the development of interstitial fibrosis, inflammation, proteinuria and hypertension. These parameters of CKD progression were significantly attenuated by immunosuppression with mycophenolate, suggesting that lymphocyte activity also modulates the AKI-to-CKD transition.

Naïve CD4+ cells differentiate into effector T-helper cells in the ischemic milieu, where they are exposed to different antigens and pro-inflammatory cytokines. T-helper cells secrete various cytokines and are thought to orchestrate the adaptive immune response. It was demonstrated that T-helper 17 (Th17) cells, which secrete the cytokine IL17, are the prominent lymphocyte population found in rat kidney following I/R injury. These cells have been implicated in a variety of autoimmune diseases such as asthma, psoriasis, inflammatory bowel disease and lupus erythematosus. There is a significant expansion of Th17 cells in kidney within the first 3 days of I/R injury in rats, while Th17 levels resolve to near sham-operated control values within 7 days as renal function recovers. However, subsequent exposure of rats to high salt diet (4%) strongly reactivates Th17 cell expression in post-ischemic kidney. This re-activation may contribute to CKD, since an IL17R antagonist attenuated renal interstitial fibrosis and neutrophil infiltration in post I/R rats exposed to high salt diet.

The basis for activation of Th17 cells in response to renal injury and exposure to high-salt diet remains to be elucidated. Th17 cell differentiation is dependent on the activity of the transcription factor RORγT and inhibitors of this factor can alleviate the pathological activation of Th17 cells. Activation of these cells by high salt diet has also been demonstrated in a mouse model of autoimmune encephalitis and associated with the activity of serum and glucocorticoid regulated kinase (SGK-1) and nuclear factor of activated T-cells 5 (NFAT5). Elevation of extracellular Na+ to 170 mM enhanced differentiation from naïve CD4+ cells to Th17 cells in vitro in a process dependent SGK-1.

Using an *in vitro* stimulation assay, it was demonstrated that CD4+ T cells from post-ischemic kidney manifest enhanced expression of IL17 in response to Ang II and elevated extracellular Na+ *in vitro,* while no response was observed from sham-operated control derived CD4 cells. The mechanisms mediating the enhanced IL17 activation in CD4 cells post AKI are not known. Previous studies have also demonstrated that Orai1, the pore forming subunit of Ca²⁺ release-activated Ca²⁺ channels (CRAC), is required for Th17 cell differentiation *in vitro,* due in part to NFAT activity. Interestingly, Orai1 mutant mice, or inhibitors of Orai1 show impaired T-cell receptor (TCR) activation, reduced IL17 production and are resistant to autoimmune disorders. Therefore, the hypothesis that renal I/R enhances lymphocyte Orai1 mediated Ca²⁺ signaling that drives Th17 cell expression and, in turn, modulates AKI and AKI-to-CKD progression was tested.

Previous studies demonstrated that Th17 cells are rapidly induced following renal I/R and that IL17 contributes to AKI. To investigate the potential that Orai1 participates in AKI, Orai1 expression was measured in Th17 cells from kidneys of rats 2 days following sham or I/R injury (*Study I*)*.* Orai1 was detected in Th17 cells and the number of these cells was increased following I/R relative to sham (Fig 1A). When accounting for influx, the total number CD4+/Orai1+ cells and the number of triple-positive CD4+/IL17+/Orai1+ cells in kidney were markedly elevated by I/R injury (Figs. 1B & C). In CD4 cells, Orai1 was associated with increased IL17 signal (Figs. 1D) and IL17+ cells were found almost exclusively in Orai1+ cells in both sham and post I/R groups (Figs. 1E). Orai1 expression was also observed in CD8+, B-cells, NK cells and macrophages but the percent of these populations was modest when compared with CD4 cells (Table 5). Orai1 has 2 homologs referred to Orai2 and Orai3, which have been suggested to modulate lymphocyte responses. However, neither Orai2 nor Orai3 were significantly affected by I/R and neither Orai2+ nor Oria3+ cells co-expressed IL-17 (Fig. 7).

Kidney Th17 levels return to sham-operated control values within ~7 days of I/R. Despite the reduction of Th17 cells, Orai1 expression was maintained in CD4+ cells 7 days post I/R (Fig. 1F). Post-AKI rat kidneys also demonstrate a greater percentage of CD4 cells expressing the IL17 transcription factor, RORγT (Fig. 3A). When placed in culture, these AKI-primed CD4+ cells (7 days post I/R), but not sham CD4+ cells, increase IL17 mRNA expression following in vitro stimulation with Ang II and elevated Na⁺ (10⁻⁷M/170 mM) (Fig. 3B). This treatment also significantly increases the percentage of IL17-expressing cells from ~12% to ~49% as detected by FACS (Fig. 2A and 2B). This response specifically requires elevated Na+, since increasing osmolality to a similar degree with either mannitol or choline chloride does not induce IL17 mRNA in the presence of Ang II (Fig. 8B). The IL17+ cells induced following treatment co-express RORγT suggesting activation of a predominately Th17 phenotype (Fig. 8C).

Kidney derived CD4+ cells were examined further for markers of effector memory T cells (CD44+/CD62L-) 7 days following I/R injury. There was a ~4-fold increase in such cells from post I/R rats vs sham (1.85±0.01 % vs 7.65±1.23 %; p< 0.05). Stimulation with Ang II and elevated Na+ did not affect the percentage CD44+ effector memory T cells suggesting this population is not responsive to stimulation that promotes IL17 expression (Fig. 8D).

To evaluate a potential role for Orai1 in the IL17 response, AKI-primed CD4+ cells were stimulated with Ang II and elevated Na+ in the presence or absence of different SOCE inhibitors. Both 2-ABP and YM58483/BPT2 completely blocked the increase of IL17 mRNA as well as the increase in IL17+ cells (Figs. 2B and 2C). In addition, AnCoA4, an inhibitor considered to be highly specific for Orai1 due to its binding to stromal interaction molecule 1 (STIM1) and thereby inhibiting gating of the Orai1 channel, also completely blocked the induction of IL17 mRNA and protein.

To evaluate Orai1 activity in AKI-primed CD4+ cells further, intracellular free Ca²⁺ responses were evaluated with the fluorescent indicator fura-2. Representative tracings of sham-operated and AKI-primed CD4+ cells are shown in (Fig. 2D). When the superfusate was changed to a buffer containing Ang II and elevated Na⁺, a rapid and sustained increase in cytosolic Ca²⁺ was observed in a significant percentage of AKI-primed lymphocytes when compared to lymphocytes derived from sham-operated controls (Fig. 2D and 2E). The addition of either AnCoA4 or YM58343/BPT2 significantly attenuated the percentage of Ca²⁺-responding cells to levels similar to sham.

To evaluate if SOCE influences Th17 cells in AKI, rats were fed YM58483/BPT2 approximately 2 hours prior to 40 min of I/R (*Study II*)*.* YM58483/BPT2 significantly attenuated the level of renal injury 24 hours after reperfusion as indicated by the level of plasma creatinine (Fig. 3A) and mRNA expression of kidney injury marker-1 (Kim-1) (Fig. 3B). YM58483/2-BPT also attenuated the infiltration of total CD4+T-cells, B-cells, and dendritic cells following I/R (Figs. 3C-3F). Total IL17 expressing cells were significantly reduced by approximately ~78% in YM58483/BPT2 treated rats relative to vehicle (Figs. 3G) and this reduction of IL17+ was primarily observed in the CD4 population (Figs. 3H) while YM58483/BPT2 did not significantly effect CD8+ cells or CD8+IL17+ cells (Figs. 3D and 3I). Also, YM58483/BPT2 did not significantly influence either Th1 (IFN-γ; Figs. 3J) or Th2 (IL-4+; data not shown) cells post-ischemia.

Orai1 is present in immune cells, but may also be present in other cell types such as vascular cells. We sought to determine if the effect of YM58483/BPT on renal injury was primarily due to its effects on Th17 activation. To address this, rats were subjected to bilateral renal I/R and treated with either YM58483/BPT2, the IL17Rc receptor antagonist or both. YM58483/BPT2 treatment alone significantly reduced plasma creatinine as compared to vehicle controls in post ischemic rats, however IL-17Rc+YM58483/BPT2 treatment did not have any additional effect (Table 1). Levels of kidney CD4+ and CD4+/IL17+ cells were significantly reduced vs vehicle-treated rats by a similar degree in both YM58483/BPT and IL17Rc treated rats (Table 1). When rats were treated with a combination of YM58483/BPT and IL17Rc, the reduction in renal injury was similar to that observed with either YM58483/BPT or IL17Rc alone (Table 1). The lack of an additional effect of IL17Rc suggests that the primary effect of YM58483/BPT is due to inhibition of Th17 cells in the early post-ischemic period.

**Table 1. Effect of SOCE inhibitor YM58343 and IL17Rc on renal injury and renal lymphocyte content 24 hours following ischemia reperfusion.**

| | Sham (n=3) | I/R+Vehicle (n=6) | I/R+Ym-58343 (n=5) | I/R+IL-17Rc (n=8) | I/R+YM+IL-Rc (n=8) |
|---|---|---|---|---|---|
| Serum Creatinine (mg/dl) | 0.7±0.1 | 5.1±0.8* | 3.7±0.6 t | 3.96±1.2 | 3.9±1.0 |
| CD4+ (cells/gram) | 19590±2812 | 118964±11193* | 26156±4365 ^{†} | 19229±3175^{†} | 18636±3140^{†} |
| CD8+ cells (cells/gram) | 2446±295 | 31237±5362* | 8410±1386^{†} | 4356±559^{†} | 4929±974^{†} |
| Total IL-17+ (cells/gram) | 4117+671 | 17887+14133* | 9334+4293t | 8447+1753t | 8718+1630t |
| CD4+/IL-17+ (cells/gram) | 1937±540 | 8111±3903* | 2020±731^{†} | 1567±294^{†} | 1694±262^{†} |
| CD8+IL-17+ (cells/gram) | 30±1.1 | 1647±770* | 127±23^{†} | 251±118^{†} | 281±173^{†} |

| | | | | | |
|---|---|---|---|---|---|
| Data are mean ± S.E; * indicates p<0.05 I/R vehicle vs sham. ^{†} indicates p<0.05 vs vehicle by one-way ANOVA and Tukey's multiple comparison test. | | | | | |

To explore further whether SOCE affects Th17 differentiation and renal injury, YM58483/BPT2 was tested in a different model of AKI associated with rhabdomyolysis. Intramuscular injection of glycerol into water deprived rats resulted in severe renal injury (Table 2). In this model, renal CD8+ cells including CD8+/IL17+ cells appeared to be the predominant lymphocyte population, as opposed to the more prevalent CD4+ lymphocyte response following I/R. Nevertheless, YM58483/BPT2 significantly attenuated the rise in serum creatinine as well as the total number of IL17+ expressing cells (Table 2).

**Table 2. Effect of SOCE inhibitor YM58343 on glycerol-induced kidney injury and lymphocyte content 24 hours following injection.**

| | Glycerol+ Vehicle (n=5) | Glycerol+YM58434 (n=5) |
|---|---|---|
| Serum Creatinine (mg/dl) | 4.2 ± 0.3 | 3.3 ± 0.4* |
| CD4+ cells per g | 234294 ± 24717 | 177606 ± 282 |
| CD8+ cells per g | 552129 ± 139124 | 342433 ± 76011 |
| Total IL-17+ cells per g | 112343 ± 19217 | 60109 ± 21033* |
| CD4+/IL-17+ cells per g | 34972 ± 3108 | 20622 ± 4191* |
| CD8+/IL-17+ cells per g | 57667 ± 1820 | 19711 ± 6499* |

| | | |
|---|---|---|
| Data are mean ± S.E; * indicate p<0.05 by Student's t-test | | |

Exposure of rats to high-salt diet at 5 weeks following recovery from I/R re-stimulates renal Th17 cell activity that is thought to contribute to CKD progression. The effect of SOCE on Th17 activation by high salt diet following AKI was evaluated in rats subjected to unilateral renal I/R followed by contralateral nephrectomy and transition to high salt diet *(Study III).* Post AKI rats treated with vehicle during high salt diet treatment manifested a significant infiltration of kidney CD4+ and CD8+ cells and IL-17 expressing cells. YM58483/BPT2 treatment significantly attenuated the increase in CD4+ and CD8+ cells and the increased expression of IL17+ cells (Figs. 4A-D). YM58483/BPT2 also significantly attenuated the increase in B cells and M^{ϕ}/dendritic cells in post-AKI rats fed high salt diet (Figs 4E and 4F). Creatinine clearance at the end of the study period (i.e., 9 weeks post I/R) was significantly reduced in vehicle-treated I/R rats relative to sham-operated controls but creatinine clearance was not reduced in YM58483/BPT2 treated rats relative to sham (Figs. 4G). Post-ischemic rats treated with vehicle also showed significant alterations in other parameters related to CKD including urinary albumin excretion, the development of interstitial fibrosis, and the expression of Kim-1; these parameters were all significantly attenuated in YM58483/BPT2-treated rats (Figs. 4H and 4I and Fig. 9).

It was previously demonstrated that increased circulating Th17 cells in rat blood following I/R, suggesting that blood may be used as source of activated lymphocytes in the setting of AKI. To investigate the potential that SOCE influences Th17 differentiation in human AKI, peripheral blood samples were obtained from critically ill patients with and without AKI. Samples were collected within 24-48 hours of AKI diagnosis for AKI cases or within 24-48 hours of ICU admission for frequency-matched (age, gender, baseline eGFR) controls without AKI (Table 3).

| Table 3. Characteristics of Patients according to AKI status | | | | | |
|---|---|---|---|---|---|
| Number of patients | | Total 17 | AKI 9 | Controls 8 | P-value |
| Demographics | | | | | |
| Age, years, mean ± SD | | 63.9 ± 10.9 | 63.8 ± 12.0 | 64.1 ± 10.4 | 0.063 |
| Male, n (%) | | 11 | 7 (77.8) | 4 (50.0) | 0.232 |
| White race, n (%) | | 15 | 9 (100.0) | 6 (75.0) | 0.110 |
| BMI, kg/m², median [IQ1-IQ3] | | 26.0 [23.1-34.4] | 33.9 [24.9-35.9] | 25.9 [24.5-28.9] | 0.481 |
| Baseline kidney function | | | | | |
| eGFR, ml/min/1.73m², median [IQ1-IQ3] | | 89.0 [74.2-106.1] | 83.2 [75.4-89.0] | 100.7 [91.1-105.1] | 0.481 |
| SCr, mg/dl, median [IQ1-IQ3] | | 0.7 [0.6-0.9] | 0.9 [0.7-1.0] | 0.7 [0.6-0.8] | 0.093 |

| Comorbidity | | | | | |
|---|---|---|---|---|---|
| Charlson Comorbidity score, median [IQ1-IQ3] | | 3.0 [2.0-4.5] | 3.0 [2.0-5.0] | 3.0 [2.8-3.3] | 0.888 |
| Diabetes, n (%) | | 8 | 6 (66.7) | 2 (25.0) | 0.086 |
| Hypertension, n (%) | | 14 | 7 (77.8) | 7 (87.5) | 0.600 |
| Congestive heart failure, n (%) | | 2 | 1 (11.1) | 1 (12.5) | 0.929 |
| COPD, n (%) | | 6 | 2 (22.2) | 4 (50.0) | 0.232 |
| Liver disease, n (%) | | 2 | 1 (11.1) | 1 (12.5) | 0.929 |
| Anemia, n (%) | | 7 | 3 (33.3) | 4 (50.0) | 0.486 |
| Cancer, n (%) | | 6 | 3 (33.3) | 3 (37.5) | 0.858 |

| AKI characteristics | | | | | |
|---|---|---|---|---|---|
| Peak SCr, mg/dl, median [IQ1-IQ3] | | | 2.9 [2.5-3.7] | -- | |
| KDIGO stage of AKI | | | | | |
| | Stage 2, n (%) | | 3 (33.3) | -- | |
| | Stage 3 | | 6 (66.7) | -- | |

| Critical illness parameters | | | | | |
|---|---|---|---|---|---|
| CFB 72 hours, liters, median [IQ1-IQ3] | | 3.0 [0.8-4.3] | 3.7 [3.0-4.8] | 2.1 [0.8-3.0] | 0.139 |
| CFB ICU stay, liters, median [IQ1-IQ3] | | 8.3 [2.7-25.6] | 14.7 [6.4-35.4] | 3.6 (3.1-6.3] | 0.268 |
| Pressor or inotrope, n (%) | | 8 | 8 (88.9) | 0(0.0) | <0.001 |
| Mechanical ventilation, n (%) | | 12 | 8 (88.9) | 4 (50.0) | 0.079 |
| APACHE II score, median [IQ1-IQ3] | | 20.0 [14.5-26.0] | 26.0 [21.0-32.0] | 14.5 [12.0-17.3] | <0.001 |
| SOFA score, median [IQ1-IQ3] | | 7.0 [3.5-10.0] | 10.0 [8.0-11.0] | 3.5 [2.8-6.3] | 0.002 |

Comparisons were done using Fisher's exact test for categorical variables and Mann-Whitney U test for continuous variables.

In isolated blood mononuclear cells, the percentages of total IL17+ cells and CD4+/IL17+ cells were significantly higher in AKI patients vs non-AKI patients (Figs 5A-5C). Moreover, the percentage of Orai1 positive cells was also prominently increased from ~3% in non-AKI patients to ~30% in patients with AKI. Similar to studies in rat kidney, Th17 cells were predominantly found within Orai1 expressing cells vs Orai1-negative cells (Figs 5D-5F).

Next, it was examined whether AKI enhanced IL-17 responses in circulating human CD4 cells and if these responses depend on SOCE activity. Human CD4+ cells responded *in vitro* to elevated extracellular sodium by increasing IL-17 expression in samples from AKI patients, but not in those of patients without AKI (Fig 5G). In contrast to rat kidney CD4+ cells, human blood CD4+ cells responded to elevated extracellular Na+ alone and did not require the addition of Ang II. Importantly, the IL17 response appeared dependent on SOCE activity, since both YM58483/BPT2 and AnCoA4 inhibited this response (Fig. 5G).

A novel regulatory pathway related to the activation of lymphocytes in the setting of acute and chronic kidney injury based on the role of intracellular calcium signaling and the differentiation of Th17 cells is proposed. It was demonstrated that the store-operated Ca²⁺ channel Orai1 is prominently induced in renal T-cells in the setting of kidney injury. Moreover, blockade of this channel attenuated Th17 cell induction and renal damage in response to ischemia/reperfusion injury as well as subsequent exposure to high salt diet. Thus, Orai1 may represent a therapeutic target to attenuate AKI or immune mediated renal fibrosis and hypertension, which may occur secondary to AKI.

Th17 cells were originally described as a distinct T-helper subset which secretes the cytokine IL-17 and is a major factor in autoimmune disorders. Th17 cells play an important role in host defense. However, in models of asthma, inflammatory bowel disease, psoriasis, or autoimmune encephalitis, Th17 cells aggravate inflammation by recruitment of other immune cells (such as neutrophils), which express the IL17RA receptor. Th17 cells have also received significant attention in the setting of renal inflammatory disorders including ANCA associated vasculitis, crescentic glomerular nephritis and nephrotic syndrome. Following renal transplant, there is an increased prevalence of Th17 cells in patients with chronic allograft nephropathy.

Th17 cells have recently been examined in the setting of AKI. Studies using *II17-null* mice, suggest that Th17 cells contribute to the severity of renal injury in response to I/R or cisplatin. A biphasic Th17 response in rats was demonstrated, with an early transient phase of expression peaking between 1-3 days following injury and the second peak induced when rats are provided high-salt diet. Th17 cells were the predominant lymphocyte population activated by high salt diet while no significant effect was observed on Th1 or Th2 cells. The exposure to high salt diet exacerbates inflammation, fibrosis and hypertension and can be attenuated by mycophenolate or an IL17 antagonist. Therefore, the mechanisms mediating IL17 expression in response to I/R and high salt intake are of interest. An *in vitro* model using CD4+ cells from kidneys 7 days following I/R revealed that stimulation by Ang II and elevated extracellular sodium increased IL17 expression, while having no effect on Th1 or Th2 responses. This priming of CD4+ cells by AKI provided an opportunity to investigate altered lymphocyte signaling leading to Th17 differentiation in response to renal injury and was the basis of the current study.

Kleinewietfeld et al. investigated the potential mechanism by which Na+ may directly influence Th17 cell differentiation. These authors demonstrated that naïve T-cells, when cultured for 4 days with TGF-β and IL6, differentiate into Th17 cells and this response was potentiated elevating the concentration of Na+ in the culture media. In addition, the response was abrogated by inhibition of p38/MAPK, NFAT5 and SGK-1. It was suggested that the increase in Na+ concentration by up to an additional 40 mM might be observed in skin under high salt diet conditions and potentially influence T-cell function. Indeed, Th17 induction and severity of autoimmune encephalopathy was enhanced when mice were placed on a high salt diet.

TCR stimulation invokes an increase in intracellular Ca²⁺ via Ca²⁺-release activated Ca²⁺ channels (CRAC). The result of this activity is thought to be calcineurin mediated dephosphorylation of NFAT, which translocates to the nucleus and activates transcriptional programs. Genome wide RNAi screens helped to identify Orai1 as the pore forming subunit of CRAC channels. Activation of Orai1 is mediated by the activity of STIM1, an endoplasmic reticulum (ER) membrane spanning protein which senses Ca²⁺ depletion from the ER secondary to TCR stimulation. Interaction between STIM1 and Orai1 increases CRAC activity and results in sustained increases in intracellular Ca²⁺. Mutations in either *ORAI1* or *STIM1* result in a severe combined immunodeficiency (SCID) phenotype. For Th17 cell differentiation, TCR stimulation in combination with various other cytokines represents an important driver of differentiation, which is considered more inflammatory than Th1 or Th2 phenotypes. Interestingly, chemical library screening recently identified putative Orai1 inhibitors, which showed greater selectivity in abrogating Th17 differentiation vs Th1 or Th2 differentiation. Moreover, Orai1 inhibition reduced the nuclear accumulation of NFAT and RORγT, critical transcriptional regulators of Th17 differentiation.

Data from the current study provide compelling evidence for an essential role of Orai1 in the differentiation of Th17 cells following renal I/R. IL17+ expressing CD4+ cells are rapidly expanded following renal injury and, using FACS analysis, we show that Orai1 expression is also prominent in kidney CD4+ cells following renal injury. Measurements from circulating blood of critically ill patients with AKI also demonstrated a profound enhancement of Ora1+ IL17+ cells, indicating that this pathway is activated in human AKI.

Importantly, IL17 expression was almost exclusive to cells expressing Orai1, and was essentially absent in Oria1-negative cells. The data from the current study indicated that Orai1 is persistently expressed following the resolution of AKI (7 days post I/R). Given that the re-expression of IL17 following *in vitro* stimulation is inhibited by SOCE antagonist, the results suggest that Orai1 mediated SOCE channel is required for Th17 differentiation following I/R.

The potential ability to target IL17 has already shown promise in the setting of autoimmune disease and several therapeutic agents are in use to target this pathway in diseases such as psoriasis. IL17 could also represent a target for both acute and chronic kidney disease since both have been shown to be ameliorated by IL17 blockade or *II17* gene knockout strategies. Similarly, we confirmed that a SOCE antagonist showed a significant degree of protection using a standard model of AKI in rats induced by bilateral renal I/R and also extended this observation to a model of AKI secondary to rhabdomyolysis. The protection was associated with a clear reduction in the genesis of Th17 cells in response to injury. However, since Orai1 may be expressed in endothelial cells or smooth muscle cells, we cannot exclude the possibility that the effects observed may be independent of IL17 production. Nevertheless, in the current study, IL17Rc blockade provided no additional protection over YM58483/BPT2 alone suggesting that the primary activity of Orai1 in the early post I/R period is to promote Th17 activation.
The transition from acute to chronic kidney disease has been the subject of significant research and maladaptive repair responses predispose CKD progression. Since immune suppression strongly attenuates the AKI to CKD transition, persistent inflammation in the kidney in response to AKI represents a potential maladaptive response. It is therefore noteworthy that Orai1 expression remains persistently elevated in CD4 cells despite the recovery of kidney function and the decline in IL17 expresion. The degree and duration of sustained Orai1 expression following recovery from AKI remains unclear, but it worth noting that reactivation of Th17 cells by high salt diet was attenuated by SOCE inhibition between 35-63 days post injury. Whether Orai1 persists in activated T cells, memory T cells, or other leukocyte populations which secrete IL17 remains to be determined. An increase in effector memory T cells 7 days following I/R was demonstrated. However, this population was not affected by *in vitro* stimulation so it is not yet clear whether this population contributes directly to the IL17 response post injury. Nevertheless, we suggest that sustained Orai1 expression may represent the basis for susceptibility to re-activation of Th17 cells and therefore represents an important link predisposing to salt-sensitive CKD progression following AKI.

In addition to providing a potential link between AKI-to-CKD, there is also clear evidence that CKD predisposes to AKI. It could be suggested that sustained expression of Orai1 in CKD could enhance sensitivity to AKI, by promoting a greater inflammatory response to a given insult. It is also reasonable to suggest that increased Orai1 expression enhances the IL17 response to other inputs. For example, in the current study, IL17 expression was elevated in response to Ang II and elevated Na+ *in vitro* and was dependent on SOCE activity. Recent studies have shown that Ang II-dependent hypertension is, in part, dependent on IL17 activity; whether Orai1 modulates Ang II dependent Th17 responses in these models remains to be determined.

Taken together, the results from the current study suggest that Orai1 could be considered a novel pathway to target inflammatory renal disease associated with the Th17 phenotype. Novel inhibitors targeting this pathway are currently in development (38) and could represent therapies for inflammatory diseases associated with Th17 cells including autoimmune disease as well as AKI, CKD and salt-sensitive hypertension.

All studies used male Sprague-Dawley rats (250-300g) that were purchased from Envigo (Indianapolis, IN). Rats were anesthetized with a cocktail of ketamine (100 mg · kg⁻¹, KetoVed, Vedco Inc, St. Joseph, MO) and xylazine (50 mg · kg⁻¹, AnaSed, Lloyd Inc, Shenandoah IA). Rats were placed on a heated surgical table to maintain body temperature. A midline incision and either unilateral (left) or bilateral renal ischemia (as indicated in *Results*) was induced by applying micro-aneurism clamps on the renal pedicles for a period of 40 min. Re-establishment of perfusion was verified by visual examination following removal of the clamps. Rats were provided post-operative analgesia using buprenorphine-SR (1 mg · kg⁻¹).

Rats were allowed to recover for various periods of time as described in the *Results.* In *Study I,* lymphocytes were studied *in vitro* after recovery for either 2 or 7 days following I/R or sham surgery for evaluation of function *in vitro* or for FACS analysis. *In Study II,* the effect of SOCE on kidney injury was studied with the inhibitor YM58483/BPT2 (*N*-[4-[3,5-*Bi*s(trifluoromethyl)-1*H*-pyrazol-1-yl]phenyl]-4-methyl-1,2,3-thiadiazole-5-carboxamide; Tocris/Bio-Techne Minneapolis, MN). Rats were pretreated p.o. 2-3 hours prior to surgery with YM58483.BPT2 (1 mg • kg⁻¹) in sugar-free chocolate pudding following dilution in 100% EtOH, at, a dose previously shown to affect T cell activation in rats. In some experiments, rats were also treated with the IL17Rc (150 ng/day i.p; R&D Systems). In addition, one experiment utilized a model of rhabdomyolysis, induced by injection of 50% glycerol (10 ml/kg) into the hindlimb muscle of rats following 16 hours of water restriction.

*Study III* was designed to investigate SOCE on progression of CKD induced by high-salt diet following recovery from I/R injury using a model of AKI-to-CKD described previously. Rats acclimated to a standard diet (AIN 76A, Dyets, Bethlehem, PA) containing 0.4% NaCl were subjected to left unilateral I/R or sham surgery and allowed to recover for ~5 weeks. Rats were then subjected to right unilateral nephrectomy and subsequently exposed to elevated dietary Na⁺ (AIN76A plus 4% NaCl) for an additional 4 weeks (Fig. 9). Sham-control rats were not subjected to renal pedicle clamping, but were subjected to unilateral nephrectomy. Simultaneous with exposure to high salt diet, rats were randomly assigned to vehicle or YM58483 treatment (1 mg • kg⁻¹ • day⁻¹; p.o.). At the end of all studies, rats were deeply anesthetized with 50-100 mg/kg pentobarbital (Fatal Plus, Vortech, Dearborn, MI) and kidneys harvested for analysis.

A prospective case-control study was designed to examine peripheral blood from AKI patients (n=9) and matched-controls without AKI (n=8) admitted to the ICU at the University of Kentucky Hospital. AKI was defined by KDIGO criteria, using both serum creatinine (SCr) and urine output data. Only patients with AKI stage ≥2 were included in the study as cases. Controls were frequency-matched by age (10-year intervals), gender and 2-category baseline estimated glomerular filtration rate (eGFR, calculated using CKD EPI equation, ≥90 and 60-89 ml/min/1.73 m²). Baseline SCr was defined as the most recent SCr within the 6-month period before ICU admission. Inclusion criteria included: adults ≥18 years of age, admission to the ICU, and baseline eGFR ≥60 ml/min/1.73 m². Exclusion criteria consisted of prior kidney or any other solid organ transplant, end-stage kidney disease, evidence of AKI before ICU admission, or the presence of uroepithelial tumors.

Single-timepoint peripheral whole blood samples were obtained 24-48 hours after AKI diagnosis (cases) or ICU admission (controls). Standardized techniques for blood collection, transport and storage were employed.

To measure creatinine, tail blood was collected in heparin containing tubes and centrifuged to collect plasma. Plasma creatinine was measured using a Pointe Scientific Analyzer and Creatinine Assay reagents using methods outlined by the manufacturer (Pointe Scientific, Canton MI). Urine was collected for 24 hours by placing rats in metabolic cages and urine volume was determined gravimetrically. Urine creatinine was measured using a colorimetric assay adopted for microplate readers. Creatinine clearance was measured using U_{Cr}*V/P_{Cr}.

At the time of tissue harvest, kidneys were bisected and one half was fixed by immersion in 10% formalin, embedded in paraffin and 5 µm sections stained with picrosirus red to assess fibrosis. For quantitative analysis, five random images of renal outer medulla were obtained using Leica DMLB (Scientific Instruments, Columbus, OH) microscope with a 20x objective. The percent area of picrosirus red stain was scored in a blinded fashion using Image J (NIH).

Total RNA was obtained from kidney using Trizol and the Zymogen RNA extraction kit and cDNA was prepared using MMLV enzyme (Invitrogen, Carlsburg, CA). Quantitative real time PCR (qPCR) using gene specific primers was performed using ABI 7500 (Applied Biosystems, Foster City, CA). mRNA values were calculated using 2^{-ΔΔCt}, Specific primers sequences for IL-6 (catalog # Rn01410330_m1), Kim-1 (# Rn00597703_m1) and IL17 (Rn01757168_m1) were purchased from ThermoFisher (Waltham, MA).

Freshly harvested kidneys were minced and digested in liberase (2 µg/ml. Roche, Indianapolis IN) for 15 min at 37°C using Gentle MACs (Miltenyli, San Diego, CA). The digested tissue was filtered through a 100-µm mesh and washed with RPMI containing 10% fetal bovine serum (Invitrogen). Mononuclear cells were isolated using Percol (Sigma) density gradient centrifugation.

All antibodies, their sources and concentrations used are listed in (Table 4a and 4b).

**Table 4a. Antibodies used for flow cytometry for rat studies.**

| **Name** | **Catalog** | **Clone** | **Source** |
|---|---|---|---|
| **Mouse anti-rat CD4 PE-Cy5** | **554839** | **OX-35** | **BD Pharmingen** |
| **Mouse anti-rat CD8 Alexa fluor 647** | **561611** | **OX-8** | **BD Pharmingen** |
| **IL-17A monoclonal antibody FITC** | **11-7177-80** | **Ebio17b7** | **ebiosciences** |
| **Mouse anti-rat IFN-y FITC** | **559498** | **DB-1** | **BD Pharmingen** |
| **PE mouse anti-rat IL-4** | **555082** | **OX-81** | **BD Pharmingen** |
| **FITC Mouse Anti-Rat RT1B** | **554928** | **OX-6** | **BD Pharmingen** |
| **FITC Mouse Anti-Rat CD11b/c** | **554862** | **OX-42** | **BD Pharmingen** |
| **Anti-Orai-1** | **ACC-062** | **Peptide** | **Alomone Lab** |
| **Anti-rat CD44 APC** | **FAB6577A** | **740017** | **RnD Biosystem** |
| **Anti-Orai-2** | **ACC-061** | **Peptide** | **Alomone Lab** |
| **Anti-Orai-3** | **ACC-065** | **Peptide** | **Alomone Lab** |
| **Anti-RoRyc** | **562607** | **Q31-378** | **BD Pharmingen** |

**Table 4b. Antibodies used for flow cytometry for human studies.**

| **Name** | **Catalog** | **Clone** | **Source** |
|---|---|---|---|
| **Anti-IL-17 PE** | **512306** | **BL168** | **Biolegend** |
| **Anti-CD4 PerCp** | **317432** | **OKT4** | **Biolegend** |
| **FITC orai-1** | **ACC-060** | **Peptide** | **Alomone Labs** |

To evaluate protein expression of IL17 or Orai1, Orai2 or Orai3, cells were restimulated with PMA and ionomycin for 6 hours in the presence of monensin (Golgistop,1 µg -ml, BD Biosciences) permeabilized with saponin (10%) and stained with relevant antibody. Cells were scanned using flow cytometry (FACSCalibur, BD Biosciences, San Jose, CA) and scans were analyzed using Flowjo software (Tree Star, Ashland, OR). Lymphocyte gating strategy and a representative example of the gating strategy used in these studies is shown in Fig. 6.

CD4+T cells were isolated using the MACS Pan-T cell microbead separation kit (Miltenyl, Glabach, Germany). T cells were stimulated with plate bound anti-CD3 (precoated with 2µg/mL) and soluble anti-CD28 (1µg/mL). Cells (2.5 X 10⁵ in 0.25 ml) were incubated 12-14 hours at 37°C in RPMI medium supplemented with 10% FBS (Invitrogen) in a 48-well plate. Cells were challenged with Ang II (Sigma, 10⁻⁷M) and raising the extracellular Na+ from 140 mM to 170mM, using a 1 M NaCl solution. Calcium channel inhibitors 2ABP (10 µM, Sigma) AnCoA4 (10 µM, Millipore, Burlington, MA) and YM58483/BTP2 (10 µM) were included to evaluate effects on IL-17.

To assess Ca²⁺ responses, fura2 imaging was performed. Briefly, isolated CD4+ T cells were loaded with fura-2AM (2.5 µM, Sigma, St. Louis, MO) for 45 min, washed, placed on poly-lysine (Sigma) coated coverslips and placed in a superfusion chamber with physiological salt solution (PSS) containing 2 mM Ca²⁺. The chamber was mounted on an inverted epifluorescence microscope and signal measured with alternating excitation at 340 and 380 nm and emission at 510 nm using the InCa²⁺-imaging system (Intracellular Imaging Systems, Cincinnati, OH). Data were acquired at 1.5 Hz and representative tracings were smoothed to the 10 nearest neighbor points using GraphPad Prism. For analysis, an increase in signal intensity of > 1 standard deviation from baseline was considered a positive response. Frequency of responding cells was determined from an average 53 ± 19 cells for each animal. Cells that did not respond to the Ca²⁺ ionophore ionomycin (1 µM) at the conclusion of the study were excluded from analysis.

Fresh blood cells from patients were washed with PBS twice and stimulated with PMA, lono and monensin for 4-6 hours. Cells were stained for antibodies against IL-17, CD4 and Orai-1 (Table 5).

**Table 5. Percent of Orai1 expression in different leukocyte populations in kidney following sham and I/R injury.**

| | %Orai1+ cells | |
|---|---|---|
| | Sham | I/R |
| CD4 | 27.1±9.8 | 77.9±15.3* |
| CD8 | 0.85±0.01 | 0.59±0.02 |
| B cells | 0.98±0.48 | 1.47±0.35 |
| CD11b/c | 3.2±0.7 | 5.5±0.65 |

| | | |
|---|---|---|
| * indicates P < 0.05 I/R vs sham by Student's t-test. | | |

The samples were initially blinded and diagnosis (AKI or non AKI) revealed after measurements of all samples were completed. For in vitro stimulation of human blood cells, primary T cells were isolated from fresh blood using Straight whole blood CD4 kit from Miltneyli Biotech (Miltenyli, San Diego, CA) according to manufactures' protocol. CD4+T cells were plated at a density of 1x10⁶ cells/mL in RPMI medium supplemented with FBS. Cells were stimulated with human anti-CD3/CD28 dynabeads (Gibco; Catalogue no 11161D) along with labeled treatment overnight (~12 hours). T cells were harvested and incubated with monensin for 6 hours, prior to staining for IL-17 (Table 4).

### Study I

Lymphocytes were studied in vitro after recovery for either 2 or 7 days following I/R or sham surgery for evaluation of function in vitro or for FACS analysis. To investigate a potential role that Orai1 participates in AKI, Orai1 expression was measured in Th17 cells from kidneys of rats 2 days following sham or I/R injury. Orai1 was detected in Th17 cells and the number of these cells were increased following I/R relative to sham (Figure 1A). When accounting for influx, the total number CD4+/Orai1+ cells and the number of triple-positive CD4+/IL17+/Orai1+ cells in kidney were markedly elevated by I/R injury (Figure 1B & C). In CD4 cells, Orai1 was associated with increased IL17 signal (Figure 1D) and IL17+ cells were found almost exclusively in Oria1+ cells in both sham and post I/R groups (Figure 1E). Oria1 expression was also observed in CD8+, B-cells, NK and macrophages but the percent of these populations was modest when compared with CD4 cells (Supplemental Table 5). Orai1 has 2 homologs referred to Orai2 and Orai3, which have been suggested to modulate lymphocyte responses. However, neither Orai2 nor Orai3 were significantly affected by ischemia reperfusion and neither Oria2+ nor Oria3+ cells showed a co-expression with IL-17 (Figure 7).

To evaluate a potential role for Orai1 in the IL17 response, AKI-primed CD4+ cells were stimulated with Ang II and elevated Na+ in the presence or absence of different SOCE inhibitors. Both 2-ABP and YM58483/BPT2 completely blocked the increase IL17 mRNA (Figure 2A) as well as the increase in IL17+ cells (Figure 2C). In addition, AnCoA4, an inhibitor considered to be highly specific for Orai1 due to its binding to STIM1 (25), also completely blocked the induction of IL17 mRNA and protein.

To evaluate Orai1 activity in AKI-primed CD4+ cells further, Ca2+ responses were evaluated after loading cells with Fura-2. Representative tracings of sham-operated and AKI-primed CD4+ cells are shown in (Figure 2D). When the superfusate was changed to a buffer containing Ang II and elevated Na+, a rapid and sustained increase in cytosolic Ca2+ was observed in a significant percentage of AKI-primed lymphocytes when compared to lymphocytes derived from sham-operated controls (Fig 2D and 2E). The addition of either AnCoA4 or YM58343/BPT2 significantly attenuated the percentage of Ca2+ responding cells to levels similar to sham.

### Study II

The effect of SOCE on early ischemic kidney injury was studied with the inhibitor YM58483/BPT2 (N-[4-[3,5-Bis(trifluoromethyl)-1H-pyrazol-1-yl]phenyl]-4-methyl-1,2,3-thiadiazole-5-carboxamide; Tocris/Bio-Techne Minneapolis, MN). Rats were pretreated 2-3 hours prior to surgery, p.o. in sugar-free chocolate pudding following dilution in 100% EtOH, at 1 mg • kg-1, a dose previously shown affect T cell activation in rats. To evaluate if SOCE influences Th17 cells in AKI, rats were fed YM58483/BPT2 approximately 2 hours prior to 40 of ischemia and reperfusion. YM58483/BPT2 significantly attenuated the level of renal injury 24 hours after reperfusion as indicated by the level of plasma creatinine (Figure 3A) and mRNA expression of kidney injury marker-1 (Kim-1) (Figure 3B). YM58483/2BPT2 also attenuated the infiltration of total CD4+ and CD8+ T-cells, B-cells, and dendritic cells following I/R (Figures 3C, 3D, 3E, and 3F). Total IL17 expressing cells were significantly reduced by approximately ~ 78% in YM58483/BPT2 treated rats relative to vehicle, and the reduction was observed in both CD4 and CD8 populations (Figures 3G, 3H, and 3I). Interestingly, YM58483/BPT2 did not significantly influence either Th1 (IL-4+) or Th2 (IFNγ+) cells (Figure 3J and 3K). These data suggest the possibility that SOCE influences Th17 differentiation and the course of kidney injury in response to I/R.

As shown in Figure 8, renal injury primes IL17 mRNA response in kidney derived CD4+ cells. Renal CD4 cells were isolated from kidney 7 days following sham (open bar) or I/R surgery (black bar). Cells were incubated for 12-14 hours in media containing either 140 or 170 mM Na+ with or without Ang II (10-7M) as shown. To control for supplementation of NaCl to the media, some samples were stimulated with equimolar mannitol (60 mM) or choline chloride (30 mM) as shown. IL17 mRNA is expressed as 2-ΔΔCT and is mean ± SE from a minimum of 3 independent rats per group; * indicates P < 0.05 vs control (i.e., 140 mM Na+, no added Ang II), by one-ANOVA and Tukey's post-hoc test. Note the response of AKI primed cells with Ang II and added Na+ indicated by the arrow, which is used as the control in Figure 2C.

### Study III

Study III was designed to investigate SOCE on progression of CKD induced by high-salt diet following recovery from I/R injury using a model of AKI-to-CKD described previously. Rats acclimated to a standard diet (AIN 76A, Dyets, Bethlehem, PA) containing 0.4% NaCl were subjected left unilateral I/R or sham surgery and allowed to recover for ~5 weeks. Rats were then subjected to right unilateral nephrectomy and subsequently exposed to elevated dietary Na+ (AIN76A plus 4% NaCl) for an additional 4 weeks (Figure 9). Sham-control rats were not subjected to renal pedicle clamping, but were subjected to unilateral nephrectomy. Simultaneous with exposure to high salt diet, rats were randomly assigned to vehicle or YM58483 treatment (1 mg • kg-1 • day-1; p.o.). At the end of all studies, rats were deeply anesthetized with 50-100 mg/kg pentobarbital (Fatal Plus, Vortech, Dearborn, MI) and kidneys harvested for analysis.

In post-AKI rats, exposure to high-salt diet at 5 weeks following recovery from I/R re-stimulates renal Th17 cell activity that is thought to contribute to CKD progression. The effect of SOCE on Th17 activation by high salt diet following AKI was evaluated in rats subjected to unilateral renal I/R followed by contralateral nephrectomy and transition to high salt diet. Post AKI rats treated with vehicle during high salt diet treatment manifested a significant infiltration of kidney CD4+ cells (Figures 4A and B), IL17+ cells (including total, CD4+ and CD8+) (Figure 4J), B cells and M^{ϕ}/dendritic cells (Figures 4E and F) relative to sham-operated control animals. YM58483/BPT2 treatment significantly attenuated the re-stimulation of IL17 expressing cells as well as B-cells and DC relative to vehicle. Kidney IL6 mRNA expression was also elevated in vehicle compared with sham, and was significantly attenuated by YM58483/BPT2 (Figure 4K). Creatinine clearance at the end of the study period (i.e., 9 weeks post I/R) was significantly reduced in vehicle-treated I/R rats relative to sham-operated controls but YM58483/BPT2 treatment significantly attenuated the reduction in creatinine clearance (Figure 4G). Post-ischemic rats treated with vehicle also showed significant alterations in other parameters related to CKD including urinary albumin excretion, the development of interstitial fibrosis, and the expression of Kim-1 (Figures 4H, 4I, 4L and 9). These parameters were all significantly attenuated in YM58483/BPT2 treated rats.

All data are expressed as means ± SE or SD or median [IQ1-IQ3]. For experimental data, differences in means were established by 1-tailed Student's t-test or one-way ANOVA with Tukey's multiple comparison test as indicated in the figure legends. For clinical data, comparisons were done using Fisher's exact test for categorical variables and Mann-Whitney U test for continuous variables. Analysis was done with the aid of Graph Pad Prism software (La Jolla, CA) or SAS 9.4 (SAS institute, Cary, NC). P<0.05 was considered significant.
Rats were maintained in accordance with the policies of the National Institutes of Health *Guide for the Care and Use of Laboratory Animals.* All studies were approved by Institutional Animal Care and Use Committees at Indiana University School of Medicine, Indianapolis, IN. For human studies, the protocol was approved by the Institutional Review Board of the University of Kentucky, Lexington, KY. Informed consent was obtained for all study participants.

Various modifications and additions can be made to the embodiments disclosed herein without departing from the scope of the disclosure. For example, while the embodiments described above refer to particular features, the scope of this disclosure also includes embodiments having different combinations of features and embodiments that do not include all of the described features. Thus, the scope of the present disclosure is intended to embrace all such alternatives, modifications, and variations as fall within the scope of the claims, together with all equivalents.

All publications, patents and patent applications referenced herein are hereby incorporated by reference in their entirety for all purposes as if each such publication, patent or patent application had been individually indicated to be incorporated by reference.

The present invention, inter alia, relates to the following aspects:
1. A method to treat a renal disorder in a mammal in need thereof, the method comprising: administering to the mammal in need of treatment an effective amount of a store operated calcium entry (SOCE) inhibitor or a pharmaceutically acceptable salt thereof.
2. A method according to aspect 1, wherein the renal disorder is acute kidney injury.
3. A method according to aspect 1, wherein the renal disorder is chronic kidney disease.
4. A method according to aspect 1, wherein the renal disorder is end stage renal disease.
5. A method according to aspect 1, wherein the renal disorder is selected from the group consisting of renal inflammation, renal interstitial fibrosis, impaired renal function, proteinuria and hypertension.
6. A method according to aspect 1, wherein the renal disorder is chronic allograft nephropathy.
7. A method according to aspect 1, wherein the renal disorder is a renal inflammatory disorder and the renal inflammatory disorder is selected from the group consisting of ANCA associated vasculitis, crescentic glomerular nephritis, and nephrotic syndrome
8. A method according to aspect 1, wherein the mammal is a human.
9. A method according to aspect 1, wherein the SOCE inhibitor or pharmaceutically acceptable salt thereof is formulated as a pharmaceutical composition.
10. A method according to aspect 1, wherein the pharmaceutical composition further comprises one or more pharmaceutically acceptable carriers, diluents or excipients.
11. A method according to aspect 1, wherein the SOCE inhibitor is selected from the group consisting of 2APB, YM58483/BTP2, AnCoA4 and Orai1+ Si RNA.
12. A method to inhibit differentiation of a CD4+ T cell to a T-helper 17 (TH17) cell, comprising: administering to a mammal an effective amount of a store operated calcium entry (SOCE) inhibitor or a pharmaceutically acceptable salt thereof.
13. A method according to aspect 12, wherein the differentiation of a CD4+ T cell to a TH17 cell occurs in a kidney.
14. A method according to aspect 12, wherein the SOCE inhibitor is a compound that interferes with a mechanism by which release of calcium ions from intracellular stores is coordinated with ion influx across a plasma membrane.
15. A method to modulate store operated Ca²⁺ entry into a cell, the method comprising: administering to a mammal an effective amount of a store operated calcium entry (SOCE) inhibitor or a pharmaceutically acceptable salt thereof.
16. A method according to aspect 15, wherein the store operated Ca²⁺ entry into a cell is inhibited.
17. A method according to aspect 15, wherein the SOCE inhibitor is selective for SOC channels and does not substantially affect the activity of other types of ion channels.
18. A method to decrease an amount of pro-inflammatory cytokine IL17, the method comprising: administering to a mammal an effective amount of a store operated calcium entry (SOCE) inhibitor or a pharmaceutically acceptable salt thereof.
19. A method to decrease an amount of a Ca2+ release-activated Ca2+ channel pore forming subunit Oral1, the method comprising: administering to a mammal an effective amount of a store operated calcium entry (SOCE) inhibitor or a pharmaceutically acceptable salt thereof.
20. A method according to aspect 19, wherein the SOCE inhibitor is selective for CRAC channels and does not substantially affect the activity of other types of ion channels and/or other SOC channels.
21. Use of store operated calcium entry (SOCE) inhibitor or a pharmaceutically acceptable salt thereof for the treatment of a renal disorder in a mammal.
22. The use according to aspect 21, wherein the renal disorder is acute kidney injury.
23. The use according to aspect 21, wherein the renal disorder is chronic kidney disease.
24. The use according to aspect 21, wherein the renal disorder is end stage renal disease.
25. The use according to aspect 21, wherein the renal disorder is selected from the group consisting of renal inflammation, renal interstitial fibrosis, impaired renal function, proteinuria and hypertension.
26. The use according to aspect 21, wherein the renal disorder is chronic allograft nephropathy.
27. The use according to aspect 21, wherein the renal disorder is a renal inflammatory disorder and the renal inflammatory disorder is selected from the group consisting of ANCA associated vasculitis, crescentic glomerular nephritis, and nephrotic syndrome
28. The use according to aspect 21, wherein the mammal is a human.
29. The use according to aspect 21, wherein the SOCE inhibitor or pharmaceutically acceptable salt thereof is formulated as a pharmaceutical composition.
30. The use according to aspect 21, wherein the pharmaceutical composition further comprises one or more pharmaceutically acceptable carriers, diluents or excipients.
31. The use according to aspect 21, wherein the SOCE inhibitor is selected from the group consisting of 2APB, YM58483/BTP2, AnCoA4 and Orai1+ Si RNA.

## Claims

1. A composition for use in a method to treat a renal disorder in a mammal in need thereof, the method comprising: administering to the mammal in need thereof an effective amount of a store operated calcium entry (SOCE) inhibitor or a pharmaceutically acceptable salt thereof, wherein the SOCE inhibitor is CM4620.

2. The composition for use according to claim 1, wherein the renal disorder is acute kidney injury.

3. The composition for use according to claim 1, wherein the renal disorder is chronic kidney disease.

4. The composition for use according to claim 1, wherein the renal disorder is end stage renal disease.

5. The composition for use according to claim 1, wherein the renal disorder is selected from the group consisting of renal inflammation, renal interstitial fibrosis, impaired renal function, proteinuria and hypertension.

6. The composition for use according to claim 1, wherein the renal disorder is chronic allograft nephropathy.

7. The composition for use according to claim 1, wherein the renal disorder is a renal inflammatory disorder and the renal inflammatory disorder is selected from the group consisting of ANCA associated vasculitis, crescentic glomerular nephritis, and nephrotic syndrome.

8. The composition for use according to claim 1, wherein the mammal is a human.

9. The composition for use according to claim 1, wherein the SOCE inhibitor or pharmaceutically acceptable salt thereof is formulated as a pharmaceutical composition, wherein the pharmaceutical composition further comprises one or more pharmaceutically acceptable carriers, diluents or excipients.
